# EUROPEAN PATENT APPLICATION

(11) **EP 1 825 841 A1**
(43) Date of publication of application: **29.08.2007**
(21) Application number: 07008773.9
(22) Date of filing: 27.06.2003
(51) Int. Cl.: A61F 13/00, A61L 15/00

(54) **Antimicrobial, silver-containing wound dressing for continuous release**

(30) Priority: 28.06.2002 GB 0215023
(62) Divisional of application: 03761700.8
(71) Applicant: Bristol-Myers Squibb Company, New York, N.Y. 10154 (US)
(72) Inventor: Bowler, Phillip, Warrington Cheshire WA4 5RD (GB); Parsons, David, Heswall Wirral L60 5RN (GB); Walker, Michael, Brynford Flintshire CH8 8AX (GB)
(74) Representative: Mays, Julie

(57) **Abstract**

The invention provides for the use of an effective amount of silver in the manufacture of a wound dressing comprising an anionic polymer, which dressing, when applied to the wound, gives a controlled release of ionic silver into the wound fluid for the prevention of staining of the underlying tissue.

## Description

The present invention relates to wound dressings having antibacterial, antiviral and/or antifungal activity, to a method of producing such dressings and the use of such dressings in the treatment of wounds.

With the rise in antimicrobial resistance and a general call to reduce the use of antibiotics, silver is gaining increasing popularity as an effective antimicrobial agent. The advantage of using silver as an antimicrobial agent is that there is no formation of bacterial tolerance. This is in contrast for instance to many antibiotics. A major drawback when using ionic or metallic silver for antimicrobial purposes is however the lack of control over release of the silver ions within and from the delivery vehicle.

In the past it has been known to deliver silver ions by the use of a simple solution of silver nitrate. It is also known to deliver silver by the use of a complex with sulfadiazine. Silver sulfadiazine is used extensively in the treatment of wounds, and particularly burns, and is incorporated in a cream base and sold under the trademark Flamazine or Salvadene. As the silver is present in such products as a complex, its solubility in wound fluid is low and hence the quantity of active silver present is also low.

By contrast, if the delivery vehicle for the silver does not limit the amount of ionic silver entering the wound fluid, for example from a gauze soaked in a solution of silver nitrate, too high a concentration of silver ions is released into the wound fluid and the silver may precipitate as inactive silver compounds such as silver chloride or silver sulphide on the wound and skin. This can result in discoloration and staining of the wound and skin tissues. Such staining has been reported to give potentially permanent pigmentation of the skin, so called argyria. It is also known to deliver silver to the wound by fragmentation of metallic silver particles from a dressing. Such dressings are sold under the trademark Acticoat. These dressings may also give rise to staining of the wound, surrounding skin and other materials such as clothes or bed linen by deposition of metallic silver.

There thus exists a need for a delivery vehicle for silver ions which controls the release of silver ions to the wound fluid so that staining is minimised but an effective concentration is maintained to give the desired antimicrobial activity.

A further disadvantage of bolus delivery of silver ions into the wound fluid is that ionic silver is rapidly depleted and therefore the dressing must necessarily be frequently changed to maintain a constant presence of antimicrobial agent and minimise the opportunity for infection. This is also true of treatments which deliver very low concentrations of silver ions such as silver sulfadiazine. The repeated changing of dressings on for instance burns patients causes pain to the patient and disturbs the healing process. It may be necessary for burn wounds to be dressed for three weeks or more. There thus exists a need for a wound dressing with sustained release of silver ions which maintains an effective concentration over a prolonged wear time without the need for frequent dressing changes.

WO/02 43743A to Bristol-Myers Squibb describes the preparation of a material which contains one or more hydrophilic, amphoteric or anionic polymers, where the material has antimicrobial activity. The material is prepared by preparing a solution comprising an organic solvent and a source of silver, subjecting the polymer to the solution to incorporate a desired silver concentration into said polymer, and subjecting the polymer during or after this step to one or more agents that bind the silver to the polymer and render it photostable upon drying. The polymer is, for example, a polysaccharide and, particularly, a carboxymethylcellulose or an alginate or a mixture thereof. WO/02 43743A is not concerned with skin staining caused by silver-containing wound dressings.

We have now found that wound dressings can be prepared which give a controlled, sustained release of silver ions within the dressing and into the adjacent wound fluid to give antimicrobial activity without staining the underlying tissue.

Accordingly, the invention provides for the use of an effective amount of silver in the manufacture of a wound dressing comprising an anionic polymer, which dressing, when applied to the wound, gives a controlled release of ionic silver into the wound fluid for the prevention of staining of the underlying tissue.

The wound dressing for use in the present invention comprises an amphoteric, hydrophilic, anionic polymer such as polysaccharides or modified polysaccharides, polyvinylpyrrolidone, polyvinyl alcohols, polyvinyl ethers, polyurethanes, polyacrlyates, polyacrylamides, collagen, gelatin or mixtures thereof. In preferred embodiments, the polymers contain carboxymethycellulose (CMC) such as sodium CMC. In one embodiment the polymer can be a polysaccharide comprising a carboxymethylcellulose or alginate or a mixture of carboxymethylcellulose and alginate. In other embodiments, the polymers contain gel-forming fibres comprising sodium CMC and which can be incorporated into wound dressings such as Aquacel (ConvaTec, Skillman, NJ). The polar or ionic nature of the polymer means that the binding of positively charged silver ions (cations) is facilitated.

We have found that a desired final concentration of silver in the dry wound dressing is between about 0.1% and 20% by weight, for example. Preferably between 0.1% to 10% by weight and more preferably between 0.5% and 5% by weight of the dressing. Such concentrations can be achieved by the preparation method described in WO/02 43743A.

We have also found that a desired concentration of ionic silver released by the dressing into water is preferably less than 1.5 ppm and, more preferably, between 1.5 ppm and 0.5 ppm. Most preferably the concentration of ionic silver released by the dressing into water is about 1 ppm.

Whilst not wishing to be bound by theory, the inventors hereof believe that antimicrobial efficacy is the product of the concentration of silver ions in solution and the period of wear. The concentration of silver ions is restricted by their reaction with the components of wound fluid, most notably chloride ions. Silver chloride is very sparingly soluble and in the environment created in the wound is likely to be as low as 1 *µ*g/ml. Ionic silver has a high affinity for the polymers forming the dressing matrix. Hydration of the dressing with wound fluid causes a slow and continuous dissociation of silver ions until a steady state of equilibrium is achieved between silver ions in solution and those bound to the dressing. Gelation of the dressing polymer(s) further limits the rate at which silver ions are lost onto the wound tissue. Therefore, the concentration of silver ions in wound fluid outside of the dressing remains at or slightly below the solubility limit of silver chloride. Due to this and the absence of metallic silver, the subsequent occurrence of tissue staining is much reduced. A further effect of this continuous controlled availability and reduced rate of loss of silver ions means that the weight percent of silver in the dressing is less than would be expected to maintain effective levels of silver over the wear time of the dressing.

The silver is preferably bound to the anionic, amphoteric, hydrophilic polymer by a polar or ionic bonding mechanism and is treated with a photostabilizing agent. Suitable agents include ammonia and chlorides.

The dressing is preferably in the form of a fibrous mat of the polymer but may be in the form of woven fabric or a powder or distributed within a matrix of a hydrocolloid or acrylate adhesive. The dressing can be used as part of a larger dressing or a layer in a multi-layered dressing and need not be in direct contact with the wound.

The invention is illustrated by the following examples.

### Example 1

### Analysis of Silver-containing Dressings and treatments

A dressing for use in the invention and various commercial silver-containing dressings were analysed for various properties. The data is presented in the table below.

AQUACEL-Ag samples were prepared according to the method of WO 02 43743A.

Weight per unit area was determined by weighing a complete dressing and dividing by its measured dimensions.

Loss on drying was performed gravimetrically. A minimum of 1g of sample (or a whole dressing where possible) was placed in a tared dish, weighed, heated at 105° ± 3°C for 6 hours, allowed to cool in a desiccator and then reweighed.

Silver assay was performed using atomic absorption spectrophotometry (AAS) on a wet acid digestion. If insoluble matter was present after the digestion procedure was completed it was removed by filtration prior to assay.

Dissolution experiments were carried out using a standard tablet dissolution apparatus. Approximately 3g of each test dressing were sealed into a pre-washed and hydrated cellulose dialysis membrane bag (Sigma D-9402). This was then loosely attached to the stirring paddle of the dissolution apparatus using plastic cable ties. The sample was lowered into the receiving vessel containing 300ml of dissolution medium at 37°C. The stirring rate was set at 60rpm. The temperature was maintained at 37°C and the dissolution medium sampled (10ml) at regular intervals. The sample volume was kept at 300ml by regularly topping up with the dissolution medium. The dissolution media used were (i) Normal saline (0.9%w/v NaCl(aq)) and (ii) purified water. The free silver content of the solutions was determined directly by AAS.
Normal saline was chosen to be a simple model of wound exudate in which the naturally occurring high chloride concentration would compete for available ionic silver, attempting to precipitate it from solution as insoluble silver chloride. Water was used as an alternative medium to observe the chloride free rate of silver delivery.
Silver Assay Data

| Sample | Batch No | Dressing Weight/Area as received (mg/cm²) | LOD 6 hrs 105°C (%w/w) | Silver as received (% w/w) | Silver as received (mg/cm²) | Silver on dry weight (% w/w) |
|---|---|---|---|---|---|---|
| | | | | | | |
| AQUACEL-Ag | A4592 | 9.53 | 9.94 | 1.153 | 0.110 | 1.280 |
| Acticoat Burn | 001103A-02 | 9.63 | 2.63 | 10.088 | 0.972 | 10.361 |
| Acticoat 7 | 010320A-03 | 17.6 | 2.93 | 8.414 | 1.484 | 8.668 |
| Acticoat Absorbent (Alginate) | 010524A-04 | 14.5 | 14.76 | 8.492 | 1.230 | 9.967 |
| Acticoat Moisture Control | 010316A-01 | 64.4 | 2.39 | 1.992 | 1.282 | 2.041 |
| Actisorb Silver 220 | 0117-01 | 16.9 | 13.60 | 0.069 | 0.012 | 0.079 |
| Avance Foam | 1036163 | 61.4 | 1.50 | 0.006 | 0.004 | 0.006 |
| Flamazine Cream | 11013 | 390 (Note 1) | 72.36 | 0.298 | 1.16 (Note 2) | N/A |

| | | | | | | |
|---|---|---|---|---|---|---|
| Notes 1) Flamazine cream is a 1% silver sulphadiazine formulation with a density of 0.975g/cm³ (BB867 page 6) 2) Assuming a 4mm layer is applied per treatment as indicated in the manufacturer's instructions for use 3) LOD is an abbreviation for loss on drying | | | | | | |

The Acticoat 7 and Burn products are all based polyethylene mesh coated with silver which is sprayed on. Acticoat absorbent is an alginate based product again with sprayed on silver. Acticoat moisture control is a foam product coated with silver. Avance is a foam with a zirconium ion exchange material distributed within it. Actisorb Silver 220 is a nylon bag containing a silver impregnated charcoal cloth.

Silver Dissolution Rate into Water (Total Volume 300cm³, stirred, 37°C).
Parts per million silver in solution (ppm) (µg/ml)

| Sample | Sample Mass (g) | Assay Time (hours) | | | | | | |
|---|---|---|---|---|---|---|---|---|
| | | 1.5 | 18 | 98 | 120 | 144 | 168 | 187 |
| AQUACEL-Ag A4591 | 3.2843 | 0.0117 | - | 0.623 | 0.712 | 0.907 | 0.883 | 0.922 |
| AQUACEL-Ag A4592 | 3.0435 | 0.0439 | 0.12 | 0.44 | 0.67 | 1.16 | 0.96 | 0.688 |
| Acticoat Burn | 3.1367 | 4.56 | - | 25.00 | 31.00 | 28.90 | 30.70 | 22.6 |
| Acticoat 7 | 3.2578 | 7.17 | - | 24.90 | 34.60 | 32.30 | 33.50 | 26.3 |
| Acticoat Absorbent (Alginate) | 3.2649 | 12.8 | - | 26.2 | 30.5 | 30.8 | 30.9 | 26.6 |
| Acticoat Moisture Control (Foam) | 3.3137 | 4.06 | - | 5.3 | 9.58 | 6.73 | 7.73 | 5.40 |
| Actisorb Silver 220 | 3.3166 | 0 | - | 0 | 0.02 | 0.03 | 0 | 0.0617 |
| Avance Foam | 2.9352 | 0 | - | 0.09 | 0.05 | 0.06 | 0 | 0.0897 |
| Flamazine | 2.6419 | 0.0067 | - | 0.294 | 0.363 | 0.349 | 0.353 | 0.423 |
| Control (AQUACEL) | 3.2100 | 0 | - | 0 | 0 | 0 | 0 | 0.0093 |

These results indicate that the silver metal-based Acticoat range of dressings would deliver high levels of solubilised silver rapidly to a moist wound where it will be precipitated as silver chloride. This dose dump effect, together with any metallic silver or silver oxide (Acticoat) will be deposited in the wound bed where it may cause cytotoxic effects and skin staining.

Avance contains only trace amounts of silver. In the dissolution experiments this was found to be readily released. In a moderately exuding wound one would expect this quantity of silver to be rapidly depleted and the dressing to become ineffective in the control of microbes.

Actisorb Silver 220 delivers solubilised silver very sparingly and its is predicted that although microbial growth may be retarded within the charcoal cloth, this would have very little effect on reducing the bioburden of a wound.

The mechanism by which Flamazine works is not clearly demonstrated by these experiments. Soluble silver availability is relatively low on a weight/weight basis, but repeated application of a large dose (weight/area) as indicated in the instructions for use will increase availability as will any surface active and lypophilic effects of the base ointment. Its action will also be complemented by the antimicrobial activity of the sulphadiazine component.

### Example 2

### Whole Skin Staining

Skin staining studies were carried out using an adaptation of a Franz type horizontal glass diffusion cell as described in Dugard et al, 1984. Whole human skin and wound tissue samples (1 cm² discs) were punched out using an Osborne arch punch and placed epidermal side uppermost on the receptor chamber. The donor chamber was then gently placed on top of the receptor chamber. The whole cell was clamped together and placed into a water bath maintained at 32°C ± 1°C. The underlying whole skin was bathed in saline.

Individual silver-containing wound dressings (0.64 1 cm²) were cut out and placed onto the centre of a piece of human whole skin. 200 µl of saline was placed into the donor chamber to hydrate each dressing to mimic a moderately exuding wound. Appoximately 5-10mg of Flamazine cream was placed onto the skin and gently smoothed over the exposed skin surface by means of a small spatula. Silver nitrate solution (200 *µ*l, 0.5%) was used as the positive control and water was used as the negative control. Each cell was left for 24 hours and images were taken using a Polaroid digital microscope camera.

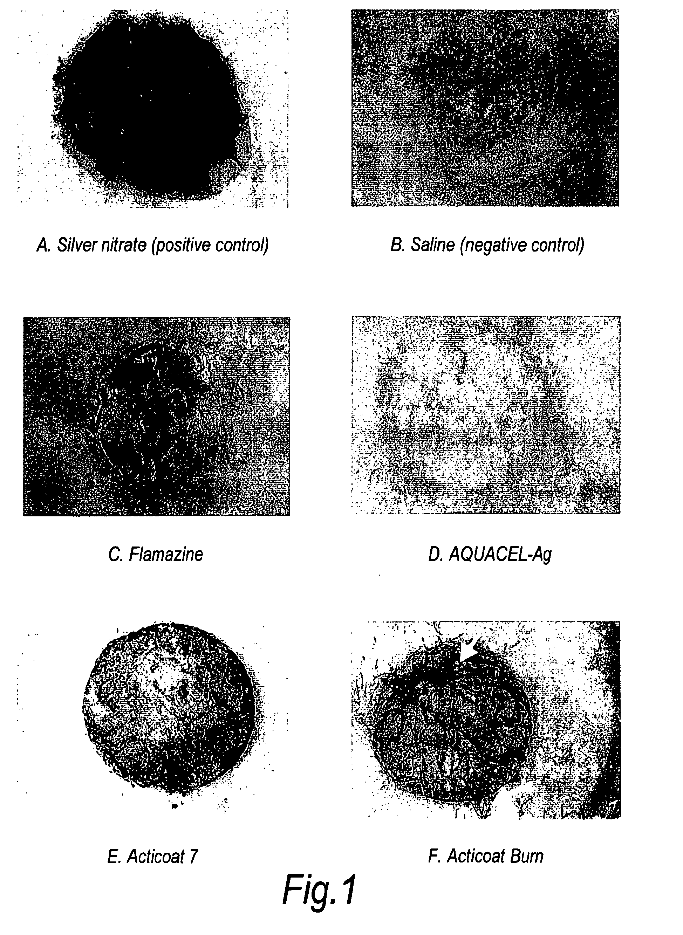

The results are presented in Figure 1. It can be seen that significant silver staining is obtained with silver nitrate, Acticoat Burn and Acticoat 7. There is minimal staining with Flamazine cream. Aquacel Ag was prepared as described in WO 02 43743A. As can be seen no skin staining was obtained by the use of Aquacel Ag according to the invention.

### Example 3

### Wound Tissue Staining Studies

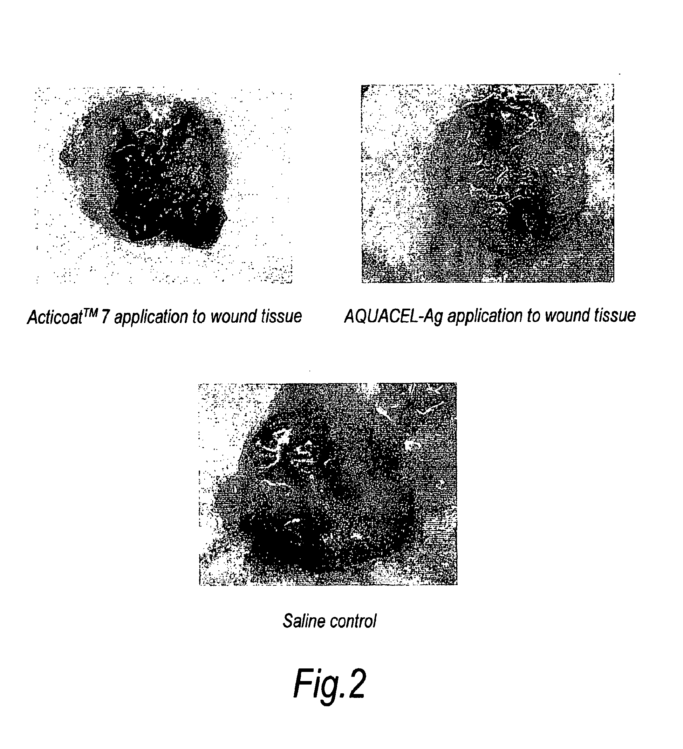

In these studies, a dressing for use in the invention, Aquacel-Ag, prepared according to WO 02 43743A, and Acticoat 7 were applied to human ulcer tissue and left in contact with the wound for 24 hours.
Saline was used as a control. The results are shown in Figure 2. The results show that no staining was obtained with use of the dressing according to the invention.

### Example 4

Dissolution experiments were performed by placing a 5x5cm piece of dressing in a vessel containing 120ml of dissolution media thermostated at 37°C. The dissolution medium was sampled (25ml) at regular intervals and the volume was kept at a constant 120ml by regularly topping up with the dissolution medium. The dissolution media used were (i) purified water and (ii) normal saline (0.9%w/v NaCl(aq)). The silver content of the sampled solutions was determined directly by AAS.
Normal saline was chosen to be a simple model of wound exudate in which the naturally occurring high chloride concentration would compete for available ionic silver, attempting to precipitate it from solution as insoluble silver chloride. Water was used as an alternative medium to observe the chloride free rate of silver delivery.
Silver Dissolution Rate.
Parts per million silver in solution (ppm) (µg/ml)

**Water**

| Time (hours) | 0 | 5 | 29 | 53 | 77 | 101 |
|---|---|---|---|---|---|---|
| AQUACEL Ag | 0.0 | 0.8 | 1.0 | 0.7 | 1.3 | 1.3 |
| Acticoat Burn | 0.0 | 35.1 | 47.3 | 33.6 | 24.9 | 21.5 |

**Normal Saline**

| Time (hours) | 0 | 4 | 24 | 48 | 72 | 96 |
|---|---|---|---|---|---|---|
| AQUACEL Ag | 0.0 | 0.9 | 0.9 | 0.9 | 0.8 | 0.7 |
| Acticoat Burn | 0.0 | 0.9 | 1.1 | 0.9 | 0.8 | 0.7 |

The difference in the ppm of silver in solution in water and in saline is an indication of the amount of silver that will be precipitated as an insoluble salt when the dressing is in use. A large difference such as that obtained for Acticoat Burn suggests that a large amount of precipitate will be produced in the wear time of the dressing. A small difference such as that obtained for Aquacel Ag suggests that a small amount of precipitate will be produced.

The results indicate that the silver metal-based Acticoat Burn dressing would deliver high levels of solubilised silver rapidly to a moist wound where it would be precipitated as silver chloride. This dose dump effect, together with any metallic silver or other insoluble silver salt will be deposited in the wound bed where it may cause cytotoxic effects and skin staining.

The results further indicate that Aquacel Ag will not deliver high levels of solubilised silver rapidly but will maintain a concentration of around 1ppm in solution throughout the wear time of the dressing.

## Claims

1. Use of an effective amount of silver in the manufacture of a wound dressing comprising an anionic, amphoteric or hydrophilic polymer, which dressing, when applied to a wound site, gives a controlled release of ionic silver into the wound fluid for the prevention of staining of the underlying tissue.

2. Use of an effective amount of silver in the manufacture of a wound dressing comprising an anionic, amphoteric or hydrophilic polymer, which, when in contact with wound exudates, gives a controlled release of ionic silver into the exudate for the maintenance of an effective antimicrobial activity within the dressing throughout the wear time of the dressing.

3. Use as claimed in any preceding claim wherein the anionic, amphoteric or hydrophilic polymer is selected from the group of polysaccharides or modified polysaccharides, polyvinylpyrrolidone, polyvinyl alcohols, polyvinyl ethers, polyurethanes, polyacrylates, polyacrylamides, collagen, gelatin or mixtures thereof.

4. Use as claimed in any preceding claim wherein the dressing is in the form of fibres, or a powder or distributed within a matrix of an adhesive.

5. Use as claimed in any preceding claim wherein the dressing comprises from 0.1 to 20% by weight of silver.

6. Use as claimed in any preceding claim wherein the release of ionic silver into water is less than 1 ppm.

7. Use as claimed in any preceding claim wherein the concentration of ionic silver released by the dressing into water is preferably less than 1.5 ppm.

8. Use as claimed by any preceding claim wherein the concentration of ionic silver released by the dressing into water is between 1.5 and 0.5 ppm.

9. Use as claimed in any preceding claim wherein the concentration of silver ions in wound fluid outside the dressing remains at or slightly below the solubility limit of silver chloride.
